# EUROPEAN PATENT SPECIFICATION

(11) **EP 0 888 162 B1**
(45) Date of publication and mention of the grant of the patent: **06.12.2000**
(21) Application number: 97902504.6
(22) Date of filing: 06.02.1997
(51) Int. Cl.: B01D 47/00, A61L 9/12, B05B 5/025

(54) **METHOD OF PRECIPITATING AIRBORNE PARTICLES**
VERFAHREN ZUR FÄLLUNG VON SCHWEBSTOFFEN
PROCEDE SERVANT A PRECIPITER DES PARTICULES EN SUSPENSION DANS L'AIR

(30) Priority: 07.02.1996 GB 9602434; 05.11.1996 GB 9622991
(43) Date of publication of application: 07.01.1999
(73) Proprietor: THE UNIVERSITY OF SOUTHAMPTON, Southampton S017 1BJ (GB); RECKITT & COLMAN PRODUCTS LIMITED, London W4 2RW (GB)
(72) Inventor: FOX, Rodney, Thomas, Hull HU16 4AS (GB); HUGHES, John, Farrell, Southampton SO40 2JL (GB); HARRISON, Neale, Mark, Staffs DE13 9HZ (GB); WHITMORE, Lindsey, Faye, Winchester, SO21 1UQ (GB)
(74) Representative: Allard, Susan Joyce
(86) International application number: GB9700353
(87) International publication number: WO9728883

(56) References cited:
- WO-A-82/01481
- US-A- 4 541 844
- US-A- 4 776 515
- US-A- 4 971 257
- DATABASE WPI Section EI, Week 8946 Derwent Publications Ltd., London, GB; Class X25, AN 89-338598 XP002028580 & SU 1 482 732 A (ALMA POWER INST) , 30 May 1989

## Description

The present invention relates to a method of precipitating airborne particles and, in particular, to a method which is suitable for domestic use.

Airborne particles such as dust aero allergens are believed to have a significant effect on individuals susceptible to allergies such as asthma. These particles reduce the air quality and can set off breathing difficulties when disturbed, for example after vacuum cleaning.

US-A-4776515 discloses an apparatus for generating a mist of negatively charged liquid drops from a capillary tube in fluid communication with a container containing the said liquid, in which an external voltage source is applied to an electrode at least partially within the capillary tube. The device operates by means of an electrodynamic pumping effect.

SU-A-1482732 discloses an apparatus for use in agriculture in which an electro aerosol sprayer is designed so that a liquid is triboelectrically charged as it flows along a tortuous path through the apparatus.

We have now developed a method of reducing the problems caused by airborne particles by causing the particles to precipitate.

Accordingly, the present invention provides a method of precipitating airborne particles from air in a domestic environment containing such particles, which method comprises contacting the airborne particles in the domestic environment with liquid droplets from a domestic aerosol spray device, a unipolar charge being imparted by double layer charging to the said liquid droplets during the actual spraying of the liquid droplets from the orifice of the aerosol spray device, the unipolar charge being at a level such that the said droplets have a charge to mass ratio of at least +/- 1 x 10⁻⁴ C/Kg, and the unipolar charge being transferred between the liquid droplets and the airborne particles by contact which causes the airborne particles to precipitate due to mutual repulsion.

Preferably the liquid droplets have a charge to mass ratio of from +/- 1 x 10⁻⁴ C/Kg to +/- 1 x 10⁻³ C/Kg. The higher the charge to mass ratio of the liquid droplets, the more pronounced the precipitation of the airborne particles.

The liquid which is sprayed from the aerosol spray device is preferably a liquid which is in the form of a water and hydrocarbon mixture, or an emulsion, or which is converted into an emulsion by shaking the aerosol device before use, or during the spraying process. Examples of domestic aerosol compositions which are in a form suitable for spraying in accordance with the method of the invention are Dettox Anti-Bacterial Room Spray and Air Wick Neutrair manufactured by Reckitt and Colman Products Ltd.

Whilst all liquid aerosols are known to carry a net negative or positive charge as a result of double layer charging, or the fragmentation of liquid droplets, the charge imparted to droplets of liquids sprayed from standard devices is only of the order of 1 x 10⁻⁸ to 1 x 10⁻⁵ C/Kg.

The invention relies on combining various characteristics of the aerosol system in order to maximise the charging of the liquid as it is sprayed from the aerosol spray device. The optimum combination varies for each product formulation.

It is possible to impart higher charges to the liquid droplets by choosing the material, shape and dimensions of the actuator, insert valve and dip tube and the characteristics of the liquid which is to be sprayed, so that the required level of charge is generated as the liquid is dispersed as droplets.

A number of characteristics of the aerosol system increase double layer charging and charge exchange between the liquid formulation and the surfaces of the aerosol system. Such increases are brought about by factors which may increase the turbulence of the flow through the system, and increase the frequency and velocity of contact between the liquid and the internal surfaces of the container and valve and actuator system.

Characteristics of the actuator can be optimised to increase the charge levels on the liquid sprayed from the container. A smaller orifice in the actuator insert, of a size of 0.45mm or less, increases the charge levels of the liquid sprayed through the actuator. The choice of material for the actuator can also increase the charge levels on the liquid sprayed from the device with materials such as nylon, PVC, and polypropylene tending to increase the charge levels. The geometry of the orifice in the insert can be optimised to increase the charge levels on the liquid as it is sprayed through the actuator. Inserts which promote the mechanical break-up of the liquid give better charging.

The actuator insert of the spray device may be formed from a conducting, insulating, semiconducting or static-dissipative material.

The characteristics of the dip tube can be optimised to increase charge levels in the liquid sprayed from the container. A narrow dip tube, of for example about 1.27mm internal diameter, increases the charge levels on the liquid, and the dip tube material can also be changed to increase charge.

Valve characteristics can be selected which increase the charge to mass ratio of the liquid product as it is sprayed from the container. A smaller tailpiece orifice in the housing, of about 0.65mm, increases product charge to mass ratio during spraying. A reduced number of holes in the stem, for example 2 x 0.50mm also increases product charge during spraying. The presence of a vapour phase tap helps to maximise the charge levels, a smaller orifice vapour phase tap, of for example about 0.50mm to 0.75mm generally giving higher charge levels.

Changes in the product formulation can also affect charging levels. A formulation containing a mixture of hydrocarbon and water, or an emulsion of an immiscible hydrocarbon and water, will carry a higher charge to mass ratio when sprayed from the aerosol device than either a water alone or hydrocarbon alone formulation.

Accordingly, in another aspect the present invention provides a method for imparting a unipolar charge to liquid droplets of a product which are sprayed from a domestic aerosol spray device at a level such that the said droplets have a charge to mass ratio of at least +/- 1 x 10⁻⁴ C/Kg, the said method comprising choosing the material of the actuator, the size of orifice of the actuator, the diameter of the dip tube and the characteristics of the valve of the domestic aerosol spray device, and the formulation of the product contained within the domestic aerosol spray device, in order to achieve the required charge to mass ratio by double layer charging imparting a unipolar charge to the droplets during the actual spraying of the liquid droplets from the orifice of the domestic aerosol spray device.

The liquid droplets sprayed from the aerosol spray device will generally have a range of droplets sizes in the range of from 5 to 100 µm, with a peak of droplets of about 40 µm.

The liquid which is sprayed from the aerosol spray device may contain a predetermined amount of a particulate material, for example fused silica, or a predetermined amount of a volatile solid material, such as menthol or naphthalene.

The method of the present invention accelerates the natural process of precipitation of airborne particles by indirect charging of the particles, thereby enabling the air quality to be improved quickly and conveniently.

The present invention enables domestic aerosol products to be used to reduce the amount of airborne dust particles in a room with effect in a short space of time. The aerosol product reduces the concentration of airborne particles in a room, making breathing easier, especially for allergy sufferers. The product would be of particular benefit directly following vacuum cleaning when the concentration of aero-allergens is elevated.

The present invention will be further described with reference to the following Example.

### EXAMPLE 1

A cloud of airborne talc particles was generated in an airtight enclosure using a powder spray gun, and the natural precipitation rate was recorded on a series of clean microscopic slides, presented for 30 seconds each, at 30 second intervals. The amount of talc on each slide was quantified by counting the number of particles in the field of view of a microscope, x140 magnification, in 10 randomly chosen locations. The mean number of particles for each 30 second interval was then calculated, and the values normalized to one to account for any variation in the cloud densities between experiments. The number of particles for the first 30 seconds after cloud generation was taken as one, and all the other values normalized to this point. The natural settling rate of talc was taken as the control.

To assess the effect of a domestic aerosol product, Airwick Neutrair (Reckitt & Colman Products Ltd) on the rate of precipitation of talc, the aerosol was sprayed into the cloud for 5 seconds, 75 seconds after generation of the cloud using three different nozzle configurations. The precipitation rate of the talc under these conditions was recorded in the same way as previously described. The natural settling rate of talc was measured as a control 1, and the procedure described was then carried out for the aerosol with a standard acetal nozzle 2 having an insert with a tapered hole, with a nozzle containing a brass insert 3 with a straight-through hole, and with a -10 kilo volts (KV) charge applied to the seam of the can from a high voltage power supply 4. Each experiment was repeated 3 times.

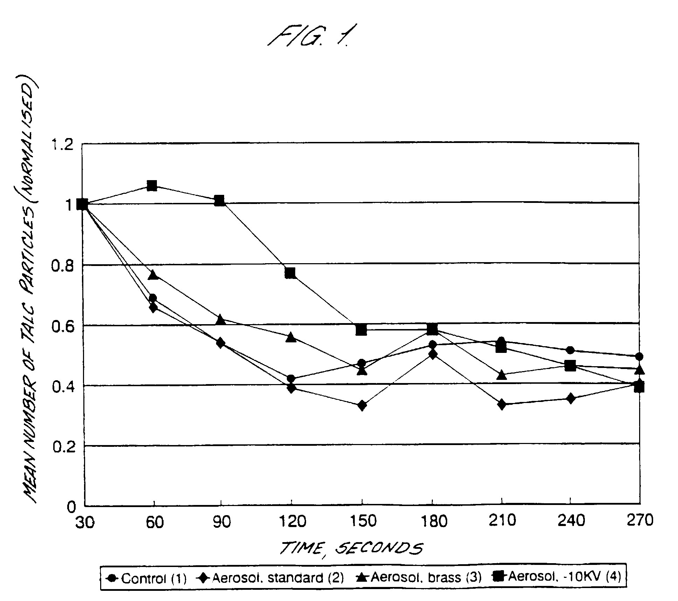

Table 1 below shows the mean number of talc particles seen in the field of view for each of the different conditions described above. The data has been normalized. This data is represented graphically in Figure 1.

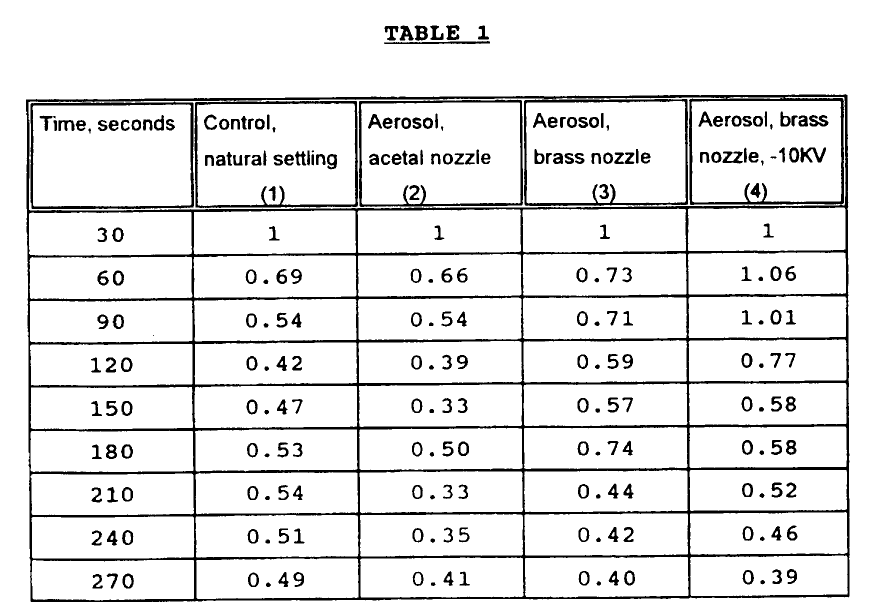

This data shows that the aerosol with the standard acetal nozzle 2 does not increase talc precipitation above the normal rate of settling 1. With the brass nozzle 3 there is a small increase in precipitation, and with the application of a high voltage to the brass nozzle 4 the charge to mass ratio of the aerosol droplets is significantly enhanced as shown in Table 2, below:

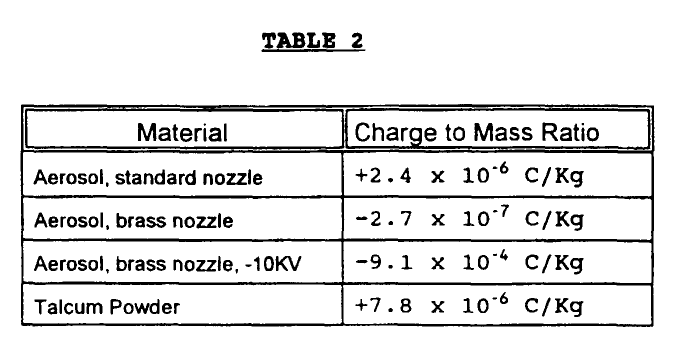

### EXAMPLE 2

A dust cloud was generated in an air tight enclosure of approximately 66m³ using 0.5 grams of house dust sieved to below 63 µm. The cloud was generated by blowing the dust for 1 second with clean, dry compressed air. The original density of the cloud 30 seconds after its generation was measured using a Malvern APC (air particle counter) 300A. The number of particles in 0.028m³ of air was recorded into 8 size bands between 1 and 25 micrometres by the particle counter. Measurements were subsequently taken 2.0, 5.5, 11.5 and 21.5 minutes after the cloud was generated. The number of particles counted into each size band at each time point was converted into the percentage of particles remaining, of those present 30 seconds after generation of the cloud. This procedure was repeated 3 times and the mean percentage for each size band at each time point calculated. This represented the natural rate of clearing the dust cloud, and was used to compare particle clearing after the various treatments.

To show the clearance rate of airborne parcels following treatment with the standard Dettox Anti-Bacterial Room Spray product (Reckitt & Colman Products Ltd), a dust cloud was generated as before. A particle count was made 30 seconds after cloud generation, and considered to be the original cloud density. 1.5 minutes after cloud generation a 0.5 second spray of the standard Dettox product was made into the dust cloud. Further particle counts were made 0.5, 4, 10 and 20 minutes after the spray of Dettox. These counts were therefore 2.0, 5.5, 11.5 and 21.5 minutes after cloud generation, and can be compared to the particle counts during natural clearing. Again, this procedure was repeated 3 times, the data corrected for liquid droplets (as described below), and converted into the percentage of particles remaining from the original cloud.

The Malvern APC 300 particle counter measures not only solid airborne particles but also airborne liquid droplets. For this reason the particle counts taken after a spray of liquid aerosol must be corrected for the number of liquid droplets originating from this source. To do this a background particle count was first taken. The aerosol was then sprayed into the enclosure for 0.5 seconds, and particle counts taken after 0.5, 4, 10 and 20 minutes. The background particle count for each size band was subtracted from the droplet count to leave the count for liquid droplets alone. Three replicates were performed. The mean number of liquid droplets in each size band, at 0.5, 4, 10 and 20 minutes after spraying the aerosol was then subtracted from the particle counts taken at these times after treatment of the dust cloud with the liquid aerosol. This leaves only the number of solid particles which remain airborne. After conversion to percentage of sold particles remaining from the original cloud, these values can be compared to the percentage of particles remaining over time during natural clearing.

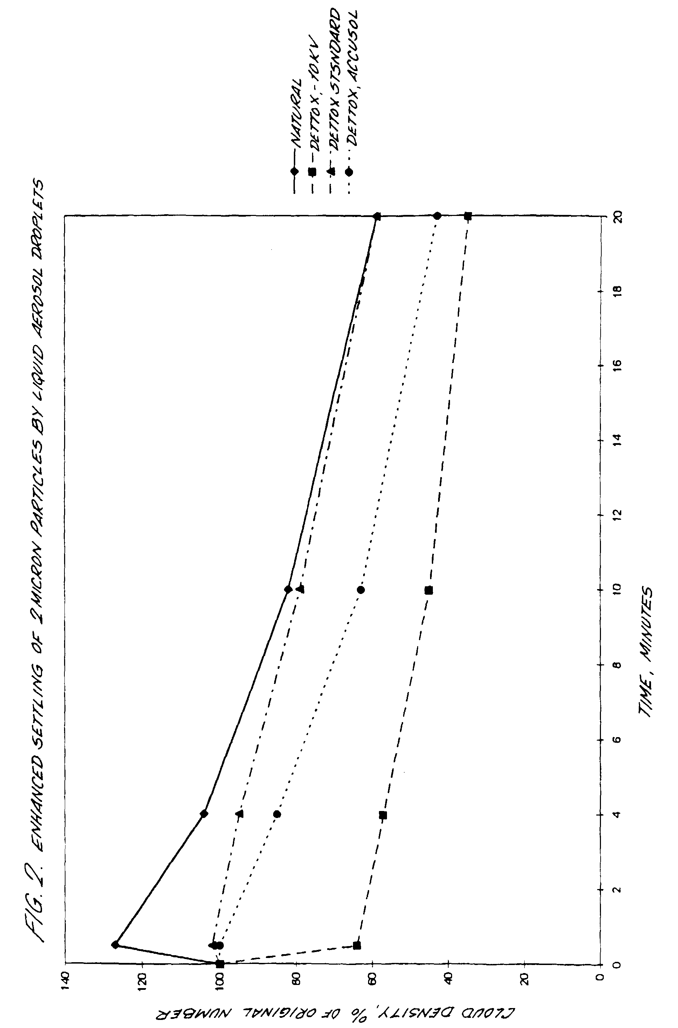

The effect of three aerosol treatments were measured in this way. The standard Dettox aerosol, Dettox with an Acc-u-sol® (manufactured by Precision Valve UK Ltd) actuator with a 0.45mm MBU CO₂ insert, and standard Dettox with -10 KV applied to the seam of the can from a high voltage power supply. The effect of the treatments on particles of between 2 and 5 micrometres is shown in Figure 2. The trend for a reduction in the number of particles remaining airborne which are of 1 to 2 µm and 5 to 25 micrometres is very similar to that shown for particles of 2 to 5 µm. Figure 2 shows that 30 seconds after spraying, for all treatments, with the exception of standard Dettox, the reduced particle concentration is maintained below the natural concentration level for more than 20 minutes. The standard Dettox product has a charge to mass ratio of 3 x 10⁻⁵ C/Kg. Treatment of a dust cloud with this product reduced the number of particles of this size remaining airborne by no more than 30%. The "Acc-u-sol" 0.45mm MBU CO actuator increases the charge to mass ratio to 6.5 x 10⁻⁵ C/Kg, and accordingly effects a slightly higher reduction in the number of airborne particles remaining. Applying -10 KV to the Dettox aerosol artificially increases the charge to mass ratio to -6.5 x 10⁻⁴ C/Kg. Figure 2 clearly shows that Dettox with this level of charge reduced the percentage of 2 to 5 micron particles remaining airborne by about 60%. This evidence demonstrates that domestic room sprays with elevated charge to mass ratios can significantly reduce the number of airborne particles in a broad spectrum of sizes.

The charge to mass ratio of domestic room sprays can be increased to the required level of 1 x 10⁻⁴ C/Kg by the methods described herein. The product Air Wick Neutrair attains a charge to mass ratio of -2 x 10⁻⁴ C/Kg when sprayed through an "Acc-u-sol" actuator with 0.45mm MBU CO₂ insert, and a valve system with 3mm (internal diameter) polyethylene dip tube, 0.65mm diameter housing orifice, 4 x 0.61mm diameter stem orifices and a 1.17mm diameter vapour phase tap.

## Claims

1. A method of precipitating airborne particles from air in a domestic environment containing such particles, which method comprises contacting the airborne particles in the domestic environment with liquid droplets from a domestic aerosol spray device, a unipolar charge being imparted by double layer charging to the said liquid droplets during the actual spraying of the liquid droplets from the orifice of the aerosol spray device, the unipolar charge being at a level such that the said droplets have a charge to mass ratio of at least +/- 1 x 10⁻⁴ C/Kg, and the unipolar charge being transferred between the liquid droplets and the airborne particles by contact which causes the airborne particles to precipitate due to mutual repulsion.

2. A method as claimed in claim 1, wherein the droplets have a charge to mass ratio of from +/- 1 x 10⁻⁴ C/Kg to +/- 1 x 10⁻³ C/Kg.

3. A method as claimed in claim 1 or claim 2 wherein the liquid droplets contain a particulate material.

4. A method as claimed in any one of the preceding claims wherein the liquid sprayed from the aerosol device is an emulsion.

5. A method as claimed in any one of the preceding claims wherein the liquid droplets have a size in the range of from 5 to 100 µm.

6. A method as claimed in any one of the preceding claims wherein the domestic aerosol spray device comprises an aerosol can.

7. A method for imparting a unipolar charge to liquid droplets of a product which are sprayed from a domestic aerosol spray device at a level such that the said droplets have a charge to mass ratio of at least +/- 1 x 10⁻⁴ C/Kg, the said method comprising choosing the material of the actuator, the size of orifice of the actuator, the diameter of the dip tube and the characteristics of the valve of the domestic aerosol spray device, and the formulation of the product contained within the domestic aerosol spray device, in order to achieve the required charge to mass ratio by double layer charging imparting a unipolar charge to the droplets during the actual spraying of the liquid droplets from the orifice of the domestic aerosol spray device.

## Patentansprüche

1. Verfahren zur Fällung von Schwebteilchen aus Luft in einer häuslichen Umgebung, welche solche Teilchen enthält, wobei das Verfahren umfaßt das in Kontakt Bringen der Schwebteilchen in der häuslichen Umgebung mit Flüssigkeitströpfchen aus einer Haushaltsaerosolsprühvorrichtung, wobei die Flüssigkeitströpfchen während des eigentlichen Versprühens der Flüssigkeitströpfchen aus der Öffnung der Aerosolsprühvorrichtung durch Doppelschichtaufladung mit einer gepolten Ladung versehen werden, die gepolte Ladung einen Wert hat, so daß die Tröpfchen ein Verhältnis von Ladung zu Masse von mindestens +/- 1 x 10⁻⁴ C/kg haben, und die gepolte Ladung zwischen den Flüssigkeitströpfchen und den Schwebteilchen durch Kontakt transferiert wird, wobei eine Fällung der Schwebteilchen aufgrund gegenseitiger Abstoßung verursacht wird.

2. Verfahren nach Anspruch 1, wobei die Tröpfchen ein Verhältnis von Ladung zu Masse von +/- 1 x 10⁻⁴ C/kg bis +/- 1 x 10⁻³ C/kg haben.

3. Verfahren nach Anspruch 1 oder Anspruch 2, wobei die Flüssigkeitströpfchen ein teilchenförmiges Material enthalten.

4. Verfahren nach einem der vorhergehenden Ansprüche, wobei die aus der Aerosolvorrichtung versprühte Flüssigkeit eine Emulsion ist.

5. Verfahren nach einem der vorhergehenden Ansprüche, wobei die Flüssigkeitströpfchen eine Größe im Bereich von 5 bis 100 µm aufweisen.

6. Verfahren nach einem der vorhergehenden Ansprüche, wobei die Haushaltsaerosolsprühvorrichtung eine Aerosoldose umfaßt.

7. Verfahren, um Flüssigkeitströpfchen eines Produkts, die aus einer Haushaltsaerosolsprühvorrichtung versprüht werden, mit einer gepolten Ladung in einem Wert zu versehen, so daß die Tröpfchen ein Verhältnis von Ladung zu Masse von mindestens +/- 1 x 10⁻⁴ C/kg haben, wobei das Verfahren umfaßt das Auswählen des Materials der Betätigungseinrichtung, der Größe der Öffnung der Betätigungseinrichtung, des Durchmessers des Tauchrohrs und der Eigenschaften des Ventils der Haushaltsaerosolsprühvorrichtung, und der Formulierung des in der Haushaltsaerosolsprühvorrichtung vorhandenen Produkts, um das erforderliche Verhältnis von Ladung zu Masse durch Doppelschichtaufladung zu erreichen, wobei die Tröpfchen während des eigentlichen Versprühens der Flüssigkeittröpfchen aus der Öffnung der Haushaltsaerosolsprühvorrichtung mit einer gepolten Ladung versehen werden.

## Revendications

1. Procédé de précipitation de particules en suspension dans l'air à partir de l'air dans un environnement domestique contenant de telles particules, lequel procédé comprend la mise en contact des particules en suspension dans l'air dans l'environnement domestique avec des gouttelettes de liquide provenant d'un dispositif de pulvérisation d'un aérosol domestique, une charge unipolaire étant communiquée par une charge en double couche desdites gouttelettes de liquide pendant la pulvérisation réelle des gouttelettes de liquide depuis l'orifice du dispositif de pulvérisation d'aérosol, la charge unipolaire étant à un niveau tel que lesdites gouttelettes ont un rapport de la charge à la masse d'au moins +/- 1 x 10⁻⁴ C/kg et la charge unipolaire étant transférée entre les gouttelettes de liquide et les particules en suspension dans l'air par contact, ce qui provoque une précipitation des particules en suspension dans l'air par suite d'une répulsion mutuelle.

2. Procédé selon la revendication 1, dans lequel les gouttelettes ont un rapport de la charge à la masse allant de +/- 1 x 10⁻⁴ C/kg à +/- 1 x 10⁻³ C/kg.

3. Procédé selon la revendication 1 ou la revendication 2, dans lequel les gouttelettes de liquide contiennent une matière en particules.

4. Procédé selon l'une quelconque des revendications précédentes, dans lequel le liquide pulvérisé depuis le dispositif à aérosol est une émulsion.

5. Procédé selon l'une quelconque des revendications précédentes, dans lequel les gouttelettes de liquide ont une dimension comprise dans la plage de 5 à 100 µm.

6. Procédé selon l'une quelconque des revendications précédentes, dans lequel le dispositif de pulvérisation d'un aérosol domestique comprend une boîte d'aérosol.

7. Procédé pour communiquer une charge unipolaire à des gouttelettes de liquide d'un produit qui sont pulvérisées depuis un dispositif de pulvérisation d'un aérosol domestique à un niveau tel que lesdites gouttelettes ont un rapport de la charge à la masse d'au moins +/- 1 x 10⁻⁴ C/kg, ledit procédé comprenant le choix de la matière de l'actionneur, de la dimension de l'orifice de l'actionneur, du diamètre du tube plongeur et des caractéristiques de la valve du dispositif de pulvérisation d'aérosol domestique, et la formulation du produit contenu dans le dispositif de pulvérisation d'aérosol domestique, de façon à parvenir au rapport demandé de la charge à la masse par une charge en double couche communiquant une charge unipolaire aux gouttelettes pendant la pulvérisation réelle des gouttelettes de liquide depuis l'orifice du dispositif de pulvérisation d'aérosol domestique.
